# EUROPEAN PATENT APPLICATION

(11) **EP 1 852 091 A1**
(43) Date of publication of application: **07.11.2007**
(21) Application number: 07251784.0
(22) Date of filing: 27.04.2007
(51) Int. Cl.: A61F 2/34, A61F 2/46

(54) **Prosthetic acetabular cup with outwardly projecting flange**

(30) Priority: 03.05.2006 GB 0608756
(71) Applicant: BENOIST GIRARD SAS, 14201 Hérouville-Saint-Clair Cédex (FR)
(72) Inventor: Ling, Robin Sydney Mackwood, Wells, Somerset, BA5 2RT (GB); Howell, Jonathan, Cullompton Devon, EX15 2NA (GB); Timperley, Andrew John, Exeter, Devon, EX5 4NW (GB); Hubble, Matthew, St. Leonard's Exeter, EX2 4NP (GB); Gie, Graham, Crediton Devon, EX17 5EZ (GB); Lavieille, Philippe, Caen, 14000 France (FR); Collet, Pascal, 14320 Saint-Martin-de-Fontenay (FR); Richard, Alain, 14610 Epron (FR)
(74) Representative: Bridge-Butler, Alan James

(57) **Abstract**

A prosthetic acetabular cup (1) having a peripheral outwardly projecting flange (10), the diametrical width (W) of which is between 4 and 6 mm.

## Description

This invention relates to a prosthetic acetabular cup which has a peripheral outwardly projecting flange.

Flanged cups are known, the flange being provided to prevent the escape of cement around the upper periphery of the cup when it is fitted. The flange also allows for the cement to be pressurized. The typical diametric width of such flanges which project from the outer surface of the cup is usually about 18 to 25 mm.

It has been found that if an accurate acetabular rim cutter is used on the bone then the outer edges of an acetabulum can be machined to provide an annular seating surface or cavity against which a flanged acetabular cup can seat.

The use of such an accurate cutter can allow for the use of a flange of much smaller overall diameter than those which have been used previously.

A flange cutter of the kind referred to above is shown in EP 0 652 482.

According to the present invention a prosthetic acetabular cup has a peripheral outwardly projecting flange, the diametrical width of which is between 4 and 6 mm. Such a flange is of a width considerably smaller than those which have been used previously.

In a preferred embodiment the diametric width is 5 mm.

Preferably the flange extends away from the mouth of the cup in a proximal direction at an angle of approximately 30°.

The inner circumference of the flange can be spaced away from the outer rim of the mouth of the cup by approximately 1.8 mm.

Preferably the flange is circular.

The outer surface of the cup can be provided with indentations to assist in adhesion with the cement and the cup can incorporate an X-ray marker.

The flange can have a thickness of approximately 1.0 mm.

The cup can be made from any suitable material, for example a synthetic plastics material, composite material, metal or a combination thereof.

The invention also includes a kit of parts comprising a prosthetic acetabular cup as set forth above and a trial cup, the dimensions of the flange of which are similar to the prosthetic acetabular cup and which include a projection or projections on its outer surface to represent a cement level in the acetabulum with which the cup is to be used.

In a preferred construction the projection is provided by an annular abutment.

The invention can be performed in various ways but one embodiment will now be described by way of example and with reference to the accompanying drawings in which :
Figure 1 is a cross-sectional elevation of a prosthetic acetabular cup, according to the present invention, on the line II-II of Figure 2;
Figure 2 is a plan view from above of the cup shown in Figure 1;
Figure 3 is a plan view from beneath of the cup shown in Figures 1 and 2;
Figure 4 shows the metallic X-ray marker which is incorporated in the cup;
Figure 5 is a side view of a trial cup for use with the cup shown in Figures 1 to 4; and,
Figure 6 shows how the cup, according to the invention, is located in an acetabulum.

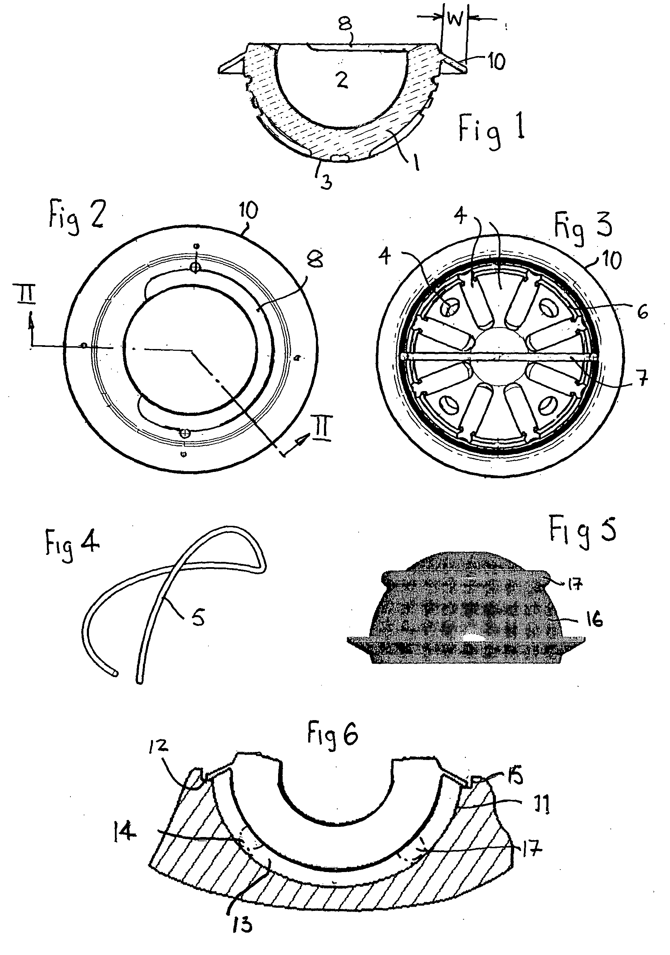

As shown in Figures 1 to 4 the prosthetic acetabular cup (1) according to the invention has a part-spherical inner bearing surface (2) and an outer surface (3) which is provided with a series of indentations and projections (4) to assist in anchoring it in the cement. The cup is provided with an X-ray marker (5), as shown in Figure 4, and which can be located in appropriate grooves (6) and (7) on the outer surface.

The mouth of the bearing surface (2) has a chamfer (8) over part of its circumference.

The cup (1) is provided with a peripheral outwardly projecting flange (10), the diametric width, which is indicated by reference letter W, is between 4 and 6 mm. In a convenient construction the width is 5 mm. The flange extends away from the mouth of the cup in a proximal direction at an angle of approximately 30° and the inner circumference of the flange is spaced away from the outer rim of the mouth of the cup by approximately 1.8 mm.

The flange is circular, as will be seen from Figures 2 and 3, and it has a thickness of approximately 1.0 mm.

Figure 6 shows how the mouth of the acetabulum (11) into which the cup is to be fitted is trimmed by machining an annular seating surface (12), as shown in Figure 6. The annular seating surface (12) is machined by an acetabular rim cutter, for example of the type shown in EP 1 652 482. The flange (10) of the acetabular cup can now be seated on the annular seating surface (12).

A gap (13) is left between the outer surface of the cup and the inner surface of the acetabulum to accept the cement. If required, spacers in the form of projections (14) could also be provided on the cup to maintain the gap (13).

The use of the rim cutter, as described above, enables an annular flange on the cup to be of much reduced diameter compared with those which have been used previously and ensures that the edge of the flange can also be located on the annular seating surface (12) which, as shown in Figure 6, can be cut into the bone to provide a cavity with an annular location rim (15). The diameter of the flange (10) can be about 1 mm less than the diameter of the location rim (15) of the cavity.The use of the small diameter flange and the cutter enables such a cut to be made which would not be possible using a large diameter flange of known type.

The invention also includes a kit of parts which comprises the cup as set forth above and a trial cup. The trial cup has dimensions and flange similar to that in the cup described above and also includes a projection or projections (17) on its outer surface to represent a cement level (13) in the acetabulum (11) with which the cup is to be used.

As will be seen from Figure 5 the projection or projections are provided by an annular abutment (17) which is also shown in chain lines in Figure 6.

The trial cup (16) is used by the surgeon to confirm the amount of cut required on the rim of the acetabulum during fitting of the cup according to the invention.

The use of the small flange guarantees an homogeneous cement mantle and it is possible to perform a reduction trial because the rim cavity ensures good stability of the cup.

Thanks to the precisely shaped flange surgeons do not need any cutting step and the diameter of the flange can correspond precisely with the rim cavity (see Figure 6). This perfect fit between the flange and the cavity leads to an optimal pressurisation of the cement.

## Claims

1. A prosthetic acetabular cup having a peripheral outwardly projecting flange, the diametrical width of which is between 4 and 6 mm.

2. A prosthetic acetabular cup as claimed in claim 1 in which the width of the flange is 5. mm

3. A prosthetic acetabular cup as claimed in claim1 in which said flange extends away from the mouth of the cup in a proximal direction at an angle of approximately 30°.

4. A prosthetic acetabular cup as claimed in claim 1, claim 2 or claim 3 in which the inner circumference of the flange is spaced away from the outer rim of the mouth of the cup by approximately 1.8 mm.

5. A prosthetic acetabular cup as claimed in claims 1 to 4 in which the flange is circular.

6. A prosthetic acetabular cup as claimed in claims 1 to 5 in which the outer surface of the cup is provided with indentations.

7. A prosthetic acetabular cup as claimed in any one pf preceding claims 1 to 5 which incorporates an X-ray marker.

8. A prosthetic acetabular cup as claimed in any one of the preceding claims in which the flange has a thickness of approximately 1.0 mm.

9. A prosthetic acetabular cup as claimed in any one of claims 1 to 8 which is intended for attachment with cement.

10. A prosthetic acetabular cup as claimed in claims 1 to 9 which is made from a synthetic plastics material, a composite material, metal or a combination thereof.

11. A kit of parts comprising a prosthetic acetabular cup as claimed in any one of the preceding claims 1 to 10 and a trial cup, the dimensions of the flange of which are similar to the prosthetic acetabular cup, and which includes a projection or projections on its outer surface to represent a cement level in the acetabulum with which the cup is to be used.

12. A kit of parts as claimed in claim 10 in which the projection is provided by an annular abutment.
